(19) Europäisches Patentamt
European Patent Office
Office européen des brevets

(11) Veröffentlichungsnummer: **0 156 769**
**B1**

(12) **EUROPÄISCHE PATENTSCHRIFT**

(45) Veröffentlichungstag der Patentschrift: **20.05.87**

(51) Int. Cl.⁴: **C 07 C 149/32**, C 07 C 148/00

(21) Anmeldenummer: **85810119.9**

(22) Anmeldetag: **20.03.85**

(54) **Verfahren zur Herstellung von Nitrodiphenyldisulfiden.**

(30) Priorität: **26.03.84 CH 1499/84**

(43) Veröffentlichungstag der Anmeldung: **02.10.85 Patentblatt 85/40**

(45) Bekanntmachung des Hinweises auf die Patenterteilung: **20.05.87 Patentblatt 87/21**

(84) Benannte Vertragsstaaten: **CH DE FR GB LI**

(56) Entgegenhaltungen:
**DE-A-3 216 126**
**DE-B-2 204 726**
**GB-A-1 222 768**

**METHODEN DER ORGANISCHEN CHEMIE, 4. Auflage, Band IX, 1955, Houben-Weyl, GEORG THIEME VERLAG, Stuttgart, Seiten 65-67**

(73) Patentinhaber: **CIBA- GEIGY AG, Klybeckstrasse 141, CH- 4002 Basel (CH)**

(72) Erfinder: **Sartori, Vittore, Dr, Erlensträsschen 65, CH- 4125 Riehen (CH)**

**EP 0 156 769 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die vorliegende Erfindung betrifft ein Verfahren zur Herstellung von Nitrodiphenyldisulfiden durch Umsetzung von Halogen-nitrobenzolen mit Alkalidisulfid.

Nitrodiphenyldisulfide sind wertvolle Zwischenprodukte, die als Ausgangsmaterial zur Herstellung verschiedener grosstechnisch wichtiger Verbindungen, wie z.B. Nitrobenzolsulfonsäure, Nitrobenzolsulfochlorid oder Orthanilsäure Verwendung finden.

Die Synthese von Nitrodiphenyldisulfiden ausgehend von Halogen-nitrobenzolen ist seit langem bekannt. H.E. FIERZ et al. geben in der Helv. Chim. Acta 663 (1929) eine Vorschrift zur Herstellung von o,o'-Dinitrodiphenyldisulfid an und zwar durch Umsetzen von o-Nitrochlorbenzol, Natriumsulfid und Schwefel in alkoholischer Lösung (siehe auch Org. Synth. Coll. Vol. I, p. 220). Die gleiche Reaktion, jedoch unter Verwendung von cyclischen oder offenkettigen Carbonsäureamiden wie z.B. N-Methylcaprolactam als Lösungsmittel ist in der DE-OS 22 04 726 beschrieben. Darüber hinaus findet man in der Literatur Verfahren zur Herstellung von 2,2',4,4'-Tetranitrodi-phenyldisulfid, verwiesen sei hier auf die Arbeiten von H.H. HODGSON et al. in J. Chem. Soc. 1002 (1948) und V.O. LUKASHEVICH et al. in Zhur. Obshchei Khim. 19, 1493 (1949) - Chem. Abstr. 44 3451i (1950). Als Reaktionsmedium wird von diesen Autoren Alkohol bzw. Akohol im Gemisch mit Wasser verwendet. Die für diese, aus dem Stand der Technik bekannten Verfahren angegebenen Ausbeuten betragen 68 bis 86 % d.Th. und liegen damit in einer für grosstechnisch hergestellte Zwischenprodukte unbefriedigenden Grössenordnung. Als ausbeutevermindernde Nebenreaktion kommt vor allem die Reaktion der Nitrogruppen, sowohl im Endprodukt, wie im Ausgangsmaterial zum tragen. Lediglich bei der gemäss DE-OS 22 04 726 in Carbonsäureamiden durchgeführten Umsetzung werden Ausbeuten von 94 % d.Th. erzielt. Die hier verwendeten Lösungsmittel sind jedoch relativ teuer und müssen am Ende der Synthese regeneriert werden.

Aufgabe der Erfindung war es somit, ein Verfahren zur Herstellung von Nitrodiphenyldisulfiden zu entwickeln, das mit einer Ausbeute von über 90 % abläuft, dabei jedoch keine aufwendig zu reinigenden Lösungsmittel benötigt.

Die Lösung dieser Aufgabe besteht darin, dass man Wasser als Reaktionsmedium verwendet und die Ausgangsverbindung, das wasserunlösliche Halogen-nitrobenzol, mit Hilfe eines Tensids darin emulgiert. Dabei zeigt sich, dass trotz des heterogenen Systems die Reaktion mit überraschend hoher Ausbeute abläuft.

Gegenstand vorliegender Erfindung ist somit ein Verfahren zur Herstellung von Nitrodiphenyldisulfiden durch Umsetzung von Halogen-nitrobenzolen der Formel

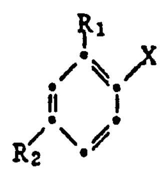

mit einem Alkalidisulfid, wobei das Verfahren dadurch gekennzeichnet ist, dass man die Reaktion in Wasser, unter Zusatz eines anionischen und/oder eines nichtionischen Tensids durchführt. Die in der Formel engegebenen Symbole haben die folgende Bedeutung: X steht für ein Chlor-, Brom- oder Jodatom; $R_1$ für Wasserstoff oder die Nitrogruppe und $R_2$ für Wasserstoff, Alkyl($C_1$-$C_4$), Halogen, die Nitro- oder Sulfogruppe, wobei zumindest einer der Reste $R_1$ oder $R_2$ die Nitrogruppe bedeutet. Unter Helogen wird Fluor, Chlor, Brom oder Jod verstanden. als Alkylreste mit 1 bis 4 C-Atomen kommen z.B. der Methyl-, Äthyl-, Propyl-, iso-Propyl- oder Butylrest in Frage.

Die Reaktion läuft nach folgender Gleichung ab:

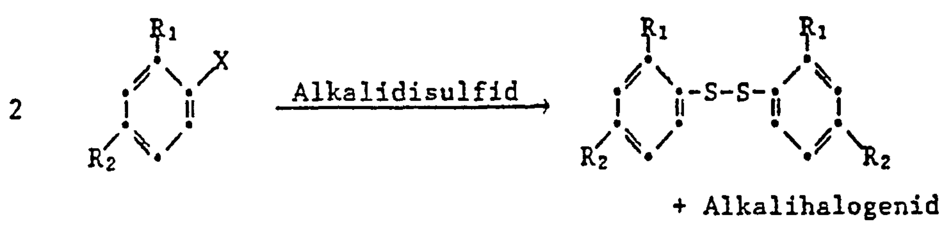


Als Halogen-nitrobenzole werden nach vorliegendem Verfahren in erster Linie das 1-Brom-2-nitrobenzol. 1-Chlor-4-nitrobenzol, 1-Brom-4-nitrobenzol, 1-Chlor-2,4-dinitrobenzol, 1-Brom-2,4-dinitro-benzol, insbesondere

jedoch das 1-Chlor-2-nitrobenzol, zu den entsprerhenden Nitrodiphenyldisulfiden umgesetzt.

Die einzelnen Halogen-nitrobenzole sind bekannt; man erhält sie beispielsweise durch Nitrierung von Halogenbenzolen [Houben-Weyl; Methoden der organischen Chemie Bd. 10/1, S. 499-515 (1971)].

Das Alkalidisulfid wird zweckmässigerweise vor der Reaktion aus Alkalihydrogensulfid und Schwefel in alkalischer Lösung frisch hergestellt. Es kann aber auch auf bekannte Art und Weise aus Alkalisulfid und Schwefel in situ gewonnen werden. Bevorzugt gelengt Natriumdisulfid zur anwendung. Das Alkalidisulfid wird vorteilhaft in geringem Überschuss eingesetzt, d.h. auf 1 Mol Halogen-nitrobenzol kommen ca. 0,55 bis 0,6 Mol Alkalidisulfid.

Bei den Tensiden, die zur bmulgierung der Halogen-nitrobenzole verwendet werden, handelt es sich um nichtionische und anionische Tenside, welche den verschiedensten Verbindungsklassen angehören können.

Als nichtionische Tenside kommen bevorzugt Äthylenoxid-Addukte von Alkylphenolen, Fettalkoholen, Fettaminen oder Fettsäuren, sog. Äthoxylate in Betracht. Das Molgewicht dieser Addukte bewegt sich zwischen 300 bis 10'000.

Als Äthylenoxid-addukte sind vor allem genannt:

a) Umsetzungsprodukte von gesättigten und/oder ungesättigten Fettalkoholen mit 10 bis 20 C-Atomen und 5 bis 40 Mol Äthylenoxid je Mol Fettalkohol;

b) Umsetzungsprodukte von Alkylphenolen mit 4 bis 12 C-Atomen im Alkylrest und 5 bis 40 Mol, vorzugsweise 5 bis 20 Mol, Äthylenoxid je Mol Alkylphenol;

c) Umsetzungsprodukte von gesättigten und/oder ungesättigten Fettaminen mit 14 bis 25 C-Atomen und 10 bis 40 Mol Äthylenozid je Mol Fettamin;

d) Umsetzungsprodukte von gesättigten und/oder ungesättigten Fettsäuren mit 14 bis 20 C-Atomen und 5 bis 40 Mol Äthylenoxid je Mol Fettsäure.

Von diesen Äthylenoxid-Addukten sind die unter d) genannten bevorzugt und zwar solche mit einem Molgewicht von 500 bis 1'500.

Auch Gemische der Äthylenoxid-Addukte nach a), b), c) und d) untereinander sind verwendbar. Diese Gemische erhält man durch Mischen einzelner Addukte oder direkt durch Äthoxylierung eines Gemisches der den Addukten zugrunde liegenden Verbindungen. Selbstverständlich kommen auch Gemische von Äthylenoxid-Addukten innerhalb einer Gruppe in Frage, wie sie beispielsweise durch Äthoxylieren einer Mischung von Fettalkoholen unterschiedlicher Rettenlänge erhalten werden.

Als gesättigte und/oder ungesättigte Fettalkohole kommen für a) Dodecanol, Palmitylalkohol, Cetylalkohol, Stearylalkohol, Oleylalkohol oder Talgfettalkohole, vorzugsweise ein Gemisch aus $C_{12}$ bis $C_{19}$-Fettalkoholen in Betracht, wie z.B. ein Gemisch aus Cetyl- und Stearylalkohol.

Als Alkylphenole für b) sind Butylphenol, Hexylphenol, vor allem jedoch Isooctylphenol, p-tert.-Octylphenol, Nonylphenol und Dodecylphenol zu nennen.

Als Fettamin für c) kommt z.B. neben Stearylamin, Palmitylamin und Oleylamin vor allem ein Gemisch von $C_{18}$ bis $C_{22}$-Fettaminen in Betracht.

Für d) sind als gesättigte und/oder ungesättigte Fettsäuren z.B. Palmitinsäure, vor allem Stearinsäure, Ricinoleinsäure und Ölsäure zu nennen.

Bei den anionischen Tensiden handelt es sich in erster Linie um Formaldehydkondensationsprodukte aromatischer Sulfonsäuren, um Alkylbenzimidazoldisulfonsäuren, deren Alkylrest 10 bis 25 C-Atome aufweist, ferner um Alkylphenolpolyglykoläthersulfate oder -phosphate mit 5 bis 15 C-Atomen im Alkylrest oder auch um Alkyl-($C_6$-$C_{20}$)-benzolsulfonate.

Eine gute emulgierende Wirkung besitzen im vorliegenden Fall auch Fettalkoholglykoläthersulfate, sowie sulfonierte oder sulfatierte Dicarbonsäureester.

Normalweise liegen die anionischen Tenside in Form ihrer Alkalisalze, ihrer Ammoniumsalze oder ihrer wasserlöslichen Aminsalze vor.

Selbstverständlich können zum Emulgieren der Halogen-nitrobenzole auch Gemische nichtionischer und anionischer Tenside verwendet werden, insondere Gemische aus einem Umsetzungsprodukt einer gesättigten und/oder ungesättigten Fettsäure mit 14 bis 20 C-Atomen und 5 bis 40 Mol Äthylenoxid bezogen auf 1 Mol Fettsäure und einem Alkyl($C_6$-$C_{20}$)-benzolsulfonat.

Bevorzugt verwendete Tenside sind auch gegebenenfalls mit Phosphorsäure veresterte Methyl ($C_4$-$C_{12}$)-phenoläthoxylate.

Eingesetzt wird das Tensid zweckmässigerweise in einer Konzentration von 2 bis 30 Gew.-%, insbesondere 5 bis 10 Gew.-%, bezogen auf Halogennitrobenzol.

Das als Reaktionsmedium verwendete Wasser wird nur in einer Menge benötigt, die etwa der des Halogen-nitrobenzols entspricht; insbesondere kommt man, gerade zu Beginn der Reaktion mit einer noch geringeren Wassermenge von 40 bis 70 Gew.- bezogen auf die Menge an Halogen-nitrobenzol aus. Diese konzentrierte Arbeitsweise - ein wesentliches Merkmal des vorliegenden Verfahren - ergibt eine hohe Raum-Zeit-Ausbeute. Durchgeführt wird die erfindungsgemäße Reaktion vorteilhaft im Temperaturbereich von 40 bis 100°C. Ausschlaggebend für die Wahl der Reaktionstemperatur ist u.a. der Schmelzpunkt des jeweils umzusetzenden Halogen-nitrobenzols.

Bei der Durchführung des Verfahrens geht man zweckmässigerweise so vor, dass man das Halogen-nitrobenzol zunächst mittels des Tensids in der entsprechenden Menge Wasser emulgiert und diese Emulsion mit der nötigen Menge an Alkalidsulfid versetzt. Um die Bildung von Nebenprodukten weitgehend zu verhindern, erweist es sich als günstig, die Alkalidsulfidlösung portionsweise oder kontinuierlich über einen

längeren Zeitraum langsam zuzugeben. Dabei kann die Dosiergeschwindigkeit über das Redoxpotential gesteuert werden; man erreicht so eine konstante Disulfidkonzentration über die gesamte Reaktionsdauer.

Eine weitere Möglichekeit besteht ferner darin, das Wasser zusammen mit dem Tensid und gegebenenfalls einem Teil des Edukts vorzulegen und das Halogen-nitrobenzol einerseits und die wässrige Disulfidlösung anderseits, synchron zuzudosieren.

Unabhängig von der Verfahrensführung wird vor allem dann ein nahezu vollständiger Umsatz der Ausgansverbindung zum Disulfid erreicht, wenn man sowohl des Edukt, als such das Produkt während der Reaktion laufend im Reaktionsmedium dispergiert. Auf diese Weise wird die Bildung von Agglomeraten aus Nitrodiphenyldisulfid und nicht verbrauchtem Edukt weitgehend vermieden. Als wirksam erweist sich hier der Einbau von Schikanen oder eines Dismembrators in den Reaktionskessel oder auch die Anwendung von Ultraschall.

Das Nitrodiphenyldisulfid fällt am Ende der Reaktion in feinkristalliner Form an und lässt sich problemlos abfiltrieren oder auch abzentrifugieren. Zweckmässigerweise wird das isolierte Produkt mit Wasser nachgewaschen.

Das erfindungsgemässe Verfahren kann beispielsweise wie folgt durchgeführt werden:

1-Chlor-2-nitrobenzol wird mit Hilfe eines nichtionischen Tenside, z.B. eines Fettsäureäthoxylats bei ca. 60°C unter intensivem Rühren in Wasser emulgiert. Dann pumpt man während mehreren Stunden kontinuierlich eine wässrige Natriumdisulfidlösung ein, die zuvor aus Natriumhydrogensulfid und Schwefel in Natronlauge frisch hergestellt wurde. Die Reaktion ist leicht exotherm. Mittels Kühlung hält man die Temperetur im Bereich von 55° bis 70°C. Anhand der Farbe des Reaktionsgemisches lässt sich der Fortgang der Reaktion leicht verfolgen. Zu Beginn ist die Reaktionsmasse rot gefärbt. Im weiteren Reaktionsverlauf scheidet sich ein grün-gelber Niederschlag ab und die wässrige Phase wird hellrot. Ist alles Natriumdisulfid zugegeben, rührt man noch für eine kurze Zeit nach, kühlt dann den Ansatz auf 30° bis 45°C herunter, filtriert das Produkt ab und wäscht mit kaltem Wasser nach. Die Ausbeute beträgt über 90 % d.Th.

Nach dem erfindungsgemässen Verfahren werden Nitrodiphenyldisulfide mit über 90%-iger Ausbeute erhalten. Das Verfahren ist einfach in der Durchführung und kostengünstig. Es zeichnet sich aus durch eine hohe Raum-Zeit-Ausbeute. Wegen der Verwendung von Wasser als Reaktionsmedium fallen keine zu regenerierenden Lösungsmittel an.

Die folgenden Beispiele dienen der Erläuterung der Erfindung; Teile bedeuten Gewichtsteile und Prozente Gewichtsprozente.

**Beispiel 1** Herstellung des Natriumdisulfids:

In einem Rührkessel werden 467 Teile Natriumhydrogensulfid als 41%-ige wässrige Lösung vorgelegt und mit 334 Teilen NaOH in Form einer 30%igen Lösung versetzt. Der Ansatz wird gelartig und durch Heizen auf 50°C wieder klar. Dann trägt man 267 Teile gemahlenen Schwefel ein und rührt bei 65 bis 70°C weiter, bis eine klare Lösung an Natriumdisulfid entsteht.

Kondensetion: 2500 Teile geschmolzenes 1-Chlor-2-nitrobenzol werden in einen Rührkessel vorgelegt und mit 150 Teilen Ricinusöläthoxylat (auf 1 Mol Ricinoleinsäure ca. 15 Mol Äthylenoxid) und 1300 Teilen Wasser versetzt. Der Ansstz wird auf 60°C erhitzt und durch intensives Rühren eine Emulsion erzeugt. Anschliessend wird die frisch hergestellte Natriumdisulfidlösung unter Niveau eingeleitet. Man gibt insgesamt 2520 Teile Disulfidlösung zu, anfangs mit einer Geschwindigkeit von ca. 300 Teilen pro Stunde und gegen Ende der Reaktion mit ca. 100 Teilen pro Stunde. Die Reaktion ist leicht exotherm; durch Kühlen wird die Temperatur in einem Bereich von 60 bis 65°C gehalten. Sich während der Reaktion bildende Klumpen aus 2,2'-Dinitrodiphenyldisulfid und 1-Chlor-2-nitrobenzol werden z.B. mittels eines Dismembrators zerkleinert. Das Reaktionsgemisch wird intensiv gerührt, um Chlornitrobenzol und gebildetes Disulfid laufend im Reaktionsmedium zu dispergieren. Nachdem die gesamte Menge an Disulfidlösung im Reaktionskessel ist, wird weitergerührt, bis die Reaktionsmasse aus einzelnen gelben Kristallen und aus farbloser bis teefarbener Mutterlauge besteht. Dann kühlt man den Ansatz auf 40°C herunter, filtriert das Produkt ab und wäscht mit kaltem Wasser nach, bis der Ablauf farblos ist. Man erhält 2897 Teile 2,2'-Dinitrodiphenyldisulfid (Gehalt 80 %), das entspricht einer Ausbeute von 95 % d.Th.; Schmelzpunkt 182°-184°C (Lit.: 196°C).

Wird anstelle des Ricinusöläthoxylats eines der folgenden Tenside verwendet, so erhält man ebenfalls eine homogene Nitrochlorbenzol-Emulsion: Nonylphenoläthoxylat (Nonylphenol/Äthylenoxid molares Verhältnis 1:9), Laurylglykoläthersulfat oder Naphthalinsulfonsäure-Formaldehydkondensat, ferner mit Phosphorsäure verestertes Nonylphenoläthoxylat oder ein sulfatierter Maleinsäureester. 2,2'-Dinitrodiphenyldisulfid ist ein wichtiges Farbstoffzwischenprodukt und dient nach exidativer Spaltung der Disulfidbrücke z.B. zur Herstellung von Säurefarbstoffen für das Färben von Polyamid Fasermaterial.

**Beispiel 2:**

1300 Teile Wasser enthaltend 150 Teile Ricinusöläthoxylat und 250 Teile 1-Chlor-2-nitrobenzol werden vorgelegt und unter intensiver Durchmischung auf 60°C erhitzt. Dann werden gleichzeitig 2268 Teile Disulfidlösung, deren Herstellung im Beispiel 1 beschrieben ist, und 2250 Teile 1-Chlor-2-nitrobenzol in getrennten Strömen zudosiert, mit einer Geschwindigkeit von 400 Teilen pro Stunde. Wenn die gesamte Menge an Chlornitrobenzol eingetragen ist, lässt man die restliche Disulfidmenge mit einer Geschwindigkeit von 100 Teilen pro Stunde zulaufen. Die Reaktion ist leicht exotherm; durch Kühlen wird die Temperatur in einem Bereich von 60 bis 65°C gehelten. Während der Reaktion wird sowohl das kontinuierlich zudosierte Chlornitrobenzol, als auch das sich bildende Disulfid fortlaufend im Reaktionsmedium dispergiert. Dazu wird die Reaktionsmasse ständig intensiv durchmischt; Agglomerate werden mit einem Dismembrator zerkleinert. Nachdem auch das Disulfid vollständig eingetragen ist, rührt man weiter, bis die Reaktionsmasse aus einzelnen gelben Kristallen und aus farbloser bis teefarbener Mutterlauge besteht. Dann kühlt man den Ansatz auf eine Temperatur von 40°C ab, filtriert und wäscht das Produkt mit kaltem Wasser, bis der Ablauf farblos ist. Man erhält ein durch ca. 5 % Nebenprodukte verunreinigtes 2,2'-Dinitrodiphenyl-disulfid in einer Ausbeute von 95 %.

**Patentansprüche**

1. Verfahren zur Herstellung von Nitrodiphenyldisulfiden der Formel

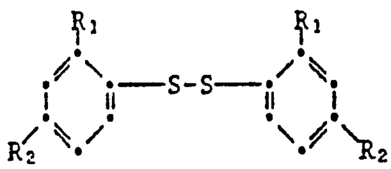

durch Umsetzung von Halogen-nitrobenzolen der Formel

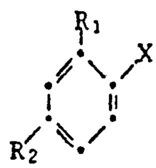

wobei X für Chlor, Brom oder Jod, $R_1$ für Wasserstoff oder Nitro und $R_2$ für Wasserstoff, Alkyl ($C_1$-$C_4$), Halogen, Nitro oder die Sulfogruppe steht, wobei zumindest einer der Reste $R_1$ oder $R_2$ Nitro bedeutet, mit einem Alkalidisulfid, dadurch gekennzeichnet, dass man die Reaktion in Wasser unter Zusatz eines nichtionischen und/oder eines anionischen Tensids durchführt.

2. Verfahren gemäss Anspruch 1, dadurch gekennzeichnet, dass man als Tensid ein nichtionisches Tensid verwendet.

3. Verfahren gemäss Anspruch 1, dadurch gekennzeichnet, dass man als Tensid ein Umsetzungsprodukt einer gesättigten und/oder ungesättigten Fettsäure mit 14 bis 20 C-Atomen und 5 bis 40 Mol Äthylenoxid bezogen auf 1 Mol Fettsäure, allein oder im Gemisch mit einem Alkyl($C_6$-$C_{20}$)-benzolsulfonat verwendet.

4. Verfahren gemäss Anspruch 1, dadurch gekennzeichnet, dass man als Tensid ein gegebenenfalls mit Phosphorsäure verestertes Alkyl ($C_4$-$C_{12}$)-phenoläthoxylat verwendet.

5. Verfahren gemäss Anspruch 1, dadurch gekennzeichnet, dass man das Tensid in einer Menge von 2 bis 30 Gew.-%, bezogen auf Halogen-nitrobenzol einsetzt.

6. Verfehren gemäss Anspruch 1, dadurch gekennzeichnet, dass man das Tensid in einer Menge von 5 bis 10 Gew.-%, bezogen auf Halogen-nitrobenzol verwendet.

7. Verfahren gemäss Anspruch 1, dadurch gekennzeichnet, dass zu Beginn der Reaktion die Wassermenge 40 bis 70 Gew.%, bezogen auf Halogen-nitrobenzol beträgt.

8. Verfahren gemäss Anspruch 1, dadurch gekennzeichnet, dass man die Reaktion bei einer Temperatur von

40° bis 100°C durchführt.

9. Verfahren gemäss Anspruch 1, dadurch gekennzeichnet, dass man die Ausgangsverbindung und das gebildete Disulfid während der Reaktion im Reaktionsmedium dispergiert.

10. Verfahren gemäss Anspruch 1, dadurch gekennzeichnet, dass man Halogen-nitrobenzol, Wasser und Tensid vorlegt und das Alkalidisulfid als wässrige Lösung zudosiert.

11. Verfehren gemäss Anspruch 1, dadurch gekennzeichnet, dass man von 1-Chlor-2-nitrobenzol ausgeht.

## Claims

1. A process for the preparation of a nitrodiphenyl disulfide of the formula

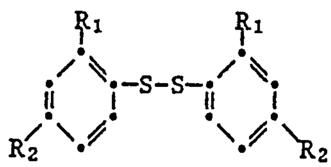

by reacting a halonitrobenzene of the formula

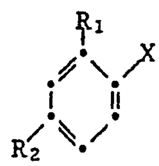

in which formulae above X is chlorine, bromine or iodine, $R_1$ is hydrogen or nitro and $R_2$ is hydrogen, $C_1$-$C_4$-alkyl, halogen, nitro or the sulfo group, with the proviso that at least one of $R_1$ and $R_2$ is nitro, with an alkali disulfide, which process comprises carrying out the reaction in water with the addition of a non-ionic and/or anionic surfactant.

2. A process according to claim 1, wherein the surfactant is a non-ionic surfactant.

3. A process according to claim 1, wherein the surfactant is an adduct of a saturated and/or unsaturated $C_{14}$-$C_{20}$fatty acid and 5 to 40 moles of ethylene oxide based on 1 mole of fatty acid, alone or in admixture with a $C_6$-$C_{20}$alkylbenzenesulfonate.

4. A process according to claim 1, wherein the surfactant is a $C_4$-$C_{12}$alkylphenol ethoxylate which may be esterified with phosphoric acid.

5. A process according to claim 1, wherein the surfactant is used in an amount of 2 to 30% by weight, based on halonitrobenzene.

6. A process according to claim 1, wherein the surfactant is used in an amount of 5 to 10% by weight, based on halonitrobenzene.

7. A process according to claim 1, wherein at the start of the reaction the amount of water is 40 to 70% by weight, based on halonitrobenzene.

8. A process according to claim 1, wherein the reaction is carried out in the temperature range from 40° to 100°C.

9. A process according to claim 1, wherein the starting compound and the resultant disulfide are dispersed in the reaction medium during the reaction.

10. A process according to claim 1, wherein the halonitrobenzene, water and the surfactant are charged to the reactor and the alkalidisulfide is added in the form of an aqueous solution.

11. A process according to claim 1, wherein the starting material is 1-chloro-2-nitrobenzene.

**Revendications**

1. Procédé pour la préparation de nitrodiphényldisulfures de formule

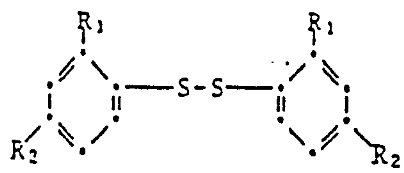

par réaction d'halogéno-nitrobenzènes de formule

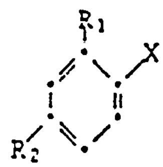

dans laquelle X représente un atome de chlore, de brome ou d'iode; $R_1$ représente un atome d'hydrogène ou un groupe nitro; et $R_2$ représente un atome d'hydrogène ou d'halogène, ou un groupe alkyle en $C_1$-$C_4$, nitro ou sulfo; au moins un des restes $R_1$ ou $R_2$ représentant un groupe nitro; avec un disulfure alcalin, caractérisé par le fait que l'on effectue la réaction dans de l'eau avec addition d'un agent tensio-actif non ionique et/ou d'un agent tensio-actif anionique.

2. Procédé selon la revendication 1, caractérisé par le fait que l'on utilise comme agent tensio-actif un agent tensio-actif non ionique.

3. Procédé selon la revendication 1, caractérisé par le fait que l'on utilise en tant qu'agent tensio-actif un produit de réaction d'un acide gras saturé et/ou d'un acide gras non saturé, ayant de 14 à 20 atomes de carbone, et de 5 à 40 moles d'oxyde d'éthylène, par rapport à une mole d'acide gras, seul ou en mélange avec un alkyl($C_6$-$C_{20}$)-benzènesulfonate.

4. Procédé selon la revendication 1, caractérisé par le fait que l'on utilise en tant qu'agent tensio-actif un alkyl($C_4$-$C_{12}$)-phénol-éthoxylate, éventuellement estérifié avec de l'acide phosphorique.

5. Procédé selon la revendication 1, caractérisé par le fait que l'on utilise l'agent tensio-actif en une quantité allant de 2 à 30% en poids, par rapport à l'halogéno-nitrobenzène.

6. Procédé selon la revendication 1, caractérisé par le fait que l'on utilise l'agent tensio-actif en une quantité allant de 5 à 10% en poids, par rapport à l'halogéno-nitrobenzène.

7. Procédé selon la revendication 1, caractérisé par le fait qu'au début de la réaction, la quantité d'eau s'élève à 40-70% en poids, par rapport à l'halogéno-nitrobenzène.

8. Procédé selon la revendication 1, caractérisé par le fait que l'on effectue la réaction à une température de 40 à 100° C.

9. Procédé selon la revendication 1, caractérisé par le fait qu'au cours de la réaction, on disperse dans le milieu réactionnel le composé de départ et le disulfure formé.

10. Procédé selon la revendication 1, caractérisé par le fait que l'on dispose au préalable l'halogéno-nitrobenzène, l'eau et l'agent tensio-actif, et on ajoute de façon réglée le disulfure alcalin, sous forme d'une solution aqueuse.

11. Procédé selon la revendication 1, caractérisé par le fait que l'on part du 1-chloro-2-nitrobenzène.